# EUROPEAN PATENT SPECIFICATION

(11) **EP 4 174 494 B1**
(45) Date of publication and mention of the grant of the patent: **19.02.2025**
(21) Application number: 21930597.6
(22) Date of filing: 27.09.2021
(51) Int. Cl.: G01N 33/80, B01L 3/00, G01N 21/07, G01N 21/75

(54) **MICROFLUIDIC BLOOD TYPE TEST CHIP**
MIKROFLUIDISCHER TESTCHIP VOM BLUTTYP
PUCE DE TEST DE TYPE SANGUIN MICROFLUIDIQUE

(30) Priority: 14.09.2021 CN 202111075012
(43) Date of publication of application: 03.05.2023
(73) Proprietor: Lansion Biotechnology Co., Ltd., Nanjing, Jiangsu 211122 (CN)
(72) Inventor: XU, Xingshang, Nanjing, Jiangsu 211122 (CN); CHEN, Jeffery, Nanjing, Jiangsu 211122 (CN); CHEN, Yabao, Nanjing, Jiangsu 211122 (CN)
(74) Representative: Zaboliene, Reda
(86) International application number: PCT/CN2021/120949
(87) International publication number: WO 2023/039938

(56) References cited:
- EP-B1- 2 349 566
- CN-A- 103 439 518
- CN-A- 104 730 258
- CN-A- 107 907 696
- CN-A- 112 169 853
- CN-U- 209 132 283
- CN-U- 211 645 203
- US-A- 5 256 376
- US-A1- 2007 077 605
- US-A1- 2007 218 515
- US-A1- 2008 085 551
- US-A1- 2008 300 148
- US-A1- 2016 318 019

## Description

### Technical Field

The invention relates to the field of microfluidic chips, in particular to a microfluidic blood type detection chip.

### Background

Microfluidic chips are an emerging technology industry. Microfluidic detection chip technology integrates basic operation units such as sample preparation, reaction, separation, and detection in biological, chemical, and medical analysis processes into a micron-scale chip, and automatically completes the entire analysis process. It has great potential in biology, chemistry, medicine and other fields.

Blood typing is a method of classifying blood, of which the ABO and Rh blood type systems are most closely related to human blood transfusion. ABO blood type system: A blood type system divided according to the presence or absence of specific antigens (agglutinogens) A and B on the surface of red blood cells. The ABO blood type system was the first human blood type system to be discovered and determined. According to the distribution of agglutinogens A and B, blood is divided into four types: A, B, AB, and O. Type A blood has only agglutinogen A on red blood cells, and there is anti-B agglutinin in its plasma; type B blood has only agglutinogen B on red blood cells, and there is anti-A agglutinin in its plasma; type AB blood has both A and B agglutinogens on red blood cells, and there is no anti-A or anti-B agglutinin in its plasma; type a blood has no A and B agglutinogens on red blood cells, and there are both anti-A and anti-B agglutinins in its plasma. Anti-A agglutinin can agglutinate erythrocytes with agglutinogen A; anti-B agglutinin can agglutinate erythrocytes with agglutinogen B. The so-called forward typing refers to the use of standard anti-A and anti-B typing plasma to determine whether there is corresponding A antigen and/or B antigen on red blood cells. The so-called reverse typing refers to the use of standard A-type and B-type cells to determine the presence or absence of anti-A and/or anti-B agglutinins in plasma. The combination of forward and reverse typing can be used as a verification to improve the accuracy of the detection results. Rh blood type system: Blood is divided into Rh-negative and Rh-positive blood types based on the presence or absence of Rh factor. Clinically, human red blood cells contain five antigens of the Rh system, antigens D, C, c, E, and e, among which the D antigen has the strongest antigenicity. According to the presence or absence of D antigen on erythrocytes, that is, blood that lacks D antigen on erythrocytes is RhD-negative blood, and blood that has D antigen on erythrocytes is RhD-positive blood.

Since incompatible transfusion of the ABO blood type system will inevitably lead to the occurrence of hemolytic transfusion reaction symptoms (disseminated intravascular coagulation, renal failure, and even death), accurate identification of ABO blood type is crucial. In addition, although the Rh-negative rate in the Chinese population is only 0.34%, the immune phenomenon due to Rh blood type incompatibility is rare, but anti-Rh antibodies can appear in the plasma of Rh-negative people after transfusion of Rh-positive blood, so when Rh-positive blood is re-infused, agglutination will occur, resulting in a hemolytic reaction. Moreover, although there is no Rh antibody in normal human plasma, if a Rh-negative woman is pregnant with a Rh-positive fetus, once the red blood cells of the fetus enter the mother, it can also stimulate the mother to produce Rh antibodies. Even if the mother is transfused for the first time, it can also cause hemolysis. Therefore, our Ministry of Health has the following clear regulations. The blood transfusion department should check the blood transfusion application form, the blood samples of the blood recipient and the blood donor item by item; review the ABO blood type (forward and/or reverse typing) of the blood recipient and the blood donor; and routinely check the Rh blood type of the patient. Crossmatching can only be performed when all of the above are correct.

Hematocrit, abbreviated as Hct, generally refers to packed cell volume (PCV), which refers to the volume ratio of sedimented red blood cells to whole blood measured after a certain amount of anticoagulated whole blood is centrifuged. It is a simple method to indirectly reflect the number and volume of red blood cells. The clinical significance of PCV determination is basically the same as that of red blood cell count or hemoglobin determination. It is often used as an indicator for the diagnosis and classification of anemia, and can also be used as an indicator for the observation of the efficacy of polycythemia vera, clinical blood transfusion and infusion therapy. It is also the basic data for calculating the mean erythrocyte volume and mean erythrocyte hemoglobin concentration.

At present, the commonly used blood type identification methods are: cardboard method, glass slide method, test tube method, and microcolumn gel method. However, the cardboard method, glass slide method and test tube method all have the following defects: 1. the forward and reverse typing identification of blood type systems needs to be carried out separately; 2. all samples need to be pre-processed, which is time-consuming and labor-intensive; 3. it cannot be automatically detected, and the proficiency of the experimenter is required to be high; and 4. large amount of blood sample is required. In addition, although the micro-column gel method can be automated, this method has extremely high requirements on the quality of the samples to be tested, reagents and instruments, and the cost is relatively high. At the same time, the detection of hematocrit is also an important basis for blood supply, and no one has yet applied it to the blood type identification chip. US Patent: US5256376 discloses a microfluidic device related to centrifugal agglutination and detection operation for determining blood type in forward typing reaction, however, it does not allow forward and reverse blood typing simultaneously on the same chip, for a whole blood sample.

### Summary of the Invention

In order to solve the deficiencies in the prior art, the present invention provides a microfluidic blood type detection chip to overcome the defects of the existing blood type detection technology. It has the advantages of simple operation, small sample usage, automatic detection, simultaneous forward and reverse typing blood type identification, low cost, more accurate detection results, and direct detection of hematocrit while performing blood type identification, providing an important basis for blood matching and supply.

In order to realize the above-mentioned technical purpose, the present invention adopts the technical scheme described in claim 1.

Preferably, the forward typing reaction chamber includes a plurality of blood type antibody reagent reaction cavities, a forward typing quality control reaction cavity and an overflow tank; the reverse typing reaction chamber includes a plurality of blood type red blood cell reagent reaction cavities, a reverse typing quality control reaction cavity and an overflow tank.

More preferably, the plurality of blood type antibody reagent reaction cavities and the plurality of blood type red blood cell reagent reaction cavities are respectively equidistantly distributed.

A diluent is preset in the second sample injection chamber, the diluent enters the quantitative separation chamber from the second sample injection chamber.

Preferably, the reverse typing blood type identification area further comprises a sample overflow chamber, which is connected with the quantitative separation chamber, and the reaction sample exceeding the quantitative separation chamber enters the sample overflow chamber.

Preferably, the quantitative separation chamber includes a separation chamber I and a separation chamber II, the sample overflow chamber and the separation chamber I are respectively connected to the separation chamber II through different microfluidic channels, the reaction sample exceeding the separation chamber I enters the separation chamber II and the sample overflow chamber successively.

Preferably, the sample overflow chamber is connected with the vent hole that is communicated with the forward typing reaction chamber.

Preferably, the microfluidic channel between the sample injection chamber and the quantitative mixing chamber, the microfluidic channel between the quantitative mixing chamber and the forward typing reaction chamber, and the microfluidic channel between the quantitative separation chamber and the reverse typing reaction chamber are pre-filled with a solid phase change material.

More preferably, the phase change material is a single-component alkane solid phase change material.

Preferably, the chip further comprises a phase change material filling port.

More preferably, the phase change material filling port is a vent hole.

The chip further comprises a hematocrit detection tube groove, which is connected to the quantitative separation chamber and is located on the side of the quantitative separation chamber close to the forward typing blood type identification area.

Preferably, the volumes of the forward typing reaction chamber and the reverse typing reaction chamber are both 20-40 µL.

Preferably, the volume of the first sample injection chamber is 100-200 µL.

Preferably, the volume of the second sample injection chamber is 250-350 µL.

According to the above-mentioned technical scheme, the present invention has the following advantages:
The invention adopts the microfluidic chip technology, and only needs to add whole blood sample on the chip to realize automatic blood type identification. In addition, the chip has forward and reverse typing blood type identification areas, a single chip can simultaneously complete the blood type detection of forward and reverse typing, and the forward and reverse typing results can be mutually verified, making the blood type identification results more accurate. Re-examination with reverse typing can make up for the deficiency of forward typing, and it is easy to find and correct the wrong identification of blood type when the results of forward and reverse typing are inconsistent.

In the present invention, the blood collection volume is small, the volume of the first sample injection chamber is 100-200 µL, and the sample injection volume is 5-20 µL, that is, a drop of blood can be used to complete the forward typing blood type detection. Therefore, the problem of applicability for people who have difficulty in blood collection, especially for newborns, can be well solved.

The invention has a built-in hematocrit detection tube groove, which can directly detect hematocrit while performing blood type identification, and provides an important basis for blood matching and supply.

In the present invention, the microfluidic channel between the first sample injection chamber and the quantitative mixing chamber is pre-filled with a phase change material. The liquid diluent is directly encapsulated in the first sample injection chamber by using the phase change material. During blood type identification, the temperature is adjusted to a suitable range through a matching detection instrument, so that the phase change material pre-filled in the microfluidic channel will undergo a phase change due to the temperature change in instrument detection chamber. The specific process is to change from solid state to liquid state, which realizes the flow of liquid sample and reagent. The chip structure is simple and the cost is low.

In the present invention, the microfluidic channel between the quantitative mixing chamber and the forward typing reaction chamber, and the microfluidic channel between the quantitative separation chamber and the reverse typing reaction chamber are pre-filled with a phase change material. Moreover, a liquid reaction reagent is directly encapsulated in the reaction chamber by using the phase change material, thereby overcoming the problems of high cost and poor reproducibility of results caused by the use of solid reagent encapsulation. Most importantly, the integrity of blood cell reagent in the reverse typing reaction chamber can be well maintained, and the problem that the blood cell is easily broken when the blood typing red blood cell reagent is solidified is solved. Therefore, the chip structure is simplified, the cost is reduced, and the accuracy of the detection result is further improved.

### Brief Description of the Drawings

FIG. 1 is an exploded schematic diagram of the overall structure of the microfluidic blood type detection chip of the present invention;
FIG. 2 is a schematic diagram of the front structure of the chip body in the microfluidic blood type detection chip of the present invention;
FIG. 3 is a schematic diagram of the rear structure of the chip body in the microfluidic blood type detection chip of the present invention;
FIG. 4 is a schematic diagram of the three-dimensional structure of the chip body in the microfluidic blood type detection chip of the present invention;
FIG. 5 is a schematic diagram of the rear structure of the overall structure of the microfluidic blood type detection chip of the present invention;
FIG. 6 is an exploded schematic diagram of the overall structure of the chip according to another embodiment of the microfluidic blood type detection chip, not part of the present invention;
FIG. 7 is a schematic diagram of the front structure of the chip body according to another embodiment of the microfluidic blood type detection chip, not part of the present invention;
FIG. 8 is a schematic diagram of the three-dimensional structure of the chip body according to another embodiment of the microfluidic blood type detection chip, not part of the present invention;
FIG. 9 is a schematic diagram of the rear structure of the chip body according to another embodiment of the microfluidic blood type detection chip, not part of the present invention;
FIG. 10 is a schematic diagram of the rear structure of the chip according to another embodiment of the microfluidic blood type detection chip, not part of the present invention;
FIG. 11 is an exploded schematic diagram of the overall structure of the chip itself of the microfluidic blood type detection chip, not part of the present invention, which is provided with two groups of forward typing blood type identification areas;
FIG. 12 is a schematic diagram of the front structure of the chip body of the microfluidic blood type detection chip, not part of the present invention, which is provided with two groups of forward typing blood type identification areas;
FIG. 13 is a schematic diagram of the three-dimensional structure of the chip body of the microfluidic blood type detection chip, not part of the present invention, which is provided with two groups of forward typing blood type identification areas;
FIG. 14 is a schematic diagram of the rear structure of the chip body of the microfluidic blood type detection chip, not part of the present invention, which is provided with two groups of forward typing blood type identification areas;
FIG. 15 is a schematic diagram of the rear structure of the chip itself of the microfluidic blood type detection chip, not part of the present invention, which is provided with two groups of forward typing blood type identification areas.

**Description of reference numerals:**
1: chip upper layer; 2: chip body; 3: opaque film; 41: first sample injection chamber; 42: second sample injection chamber; 5: anti-interference groove; 6: positioning structure; 8: quantitative mixing chamber; 9: separation chamber I; 10: hematocrit detection tube groove; 11: sample overflow chamber; 12: separation chamber II; 13: first phase change material filling port; 14: blood type antibody reagent reaction cavity; 15: blood type red blood cell reagent reaction cavity; 16: forward typing quality control reaction cavity; 17: reverse typing quality control reaction cavity; 18: overflow tank; 191: first vent hole; 192: second vent hole; 193: third vent hole; 20: sealing film; 211: first microfluidic channel; 212: second microfluidic channel; 213: third microfluidic channel; 214: fourth microfluidic channel; 215: fifth microfluidic channel; 221: upper-layer first sample injection chamber through hole; 222: upper-layer second sample injection chamber through hole; 23: sample injection chamber window; 24: hematocrit detection tube groove window; 25: reaction chamber window; 271: first reverse flow channel; 272: second reverse flow channel.

### Detailed Description of the Invention

The technical solutions of the present invention will be described in detail below with reference to the accompanying drawings. It should be understood that the specific embodiments described herein are only used to explain the present invention, but not to limit the present invention. However, the scope of the present application is not limited by these embodiments, but is subject to the scope of the claims. In order to provide a clearer description and to enable those skilled in the art to better understand the content of the present application, each part in the figures is not necessarily drawn according to its relative size. The proportions of certain sizes and other related scales may be highlighted and thus exaggerated, and irrelevant or unimportant details are not fully drawn for the sake of simplicity.

The present invention provides a microfluidic blood type detection chip, which is manufactured by injection molding of a mold and can be used with detection equipment. The detection chip can have a fan-shaped structure, or can be designed as required.

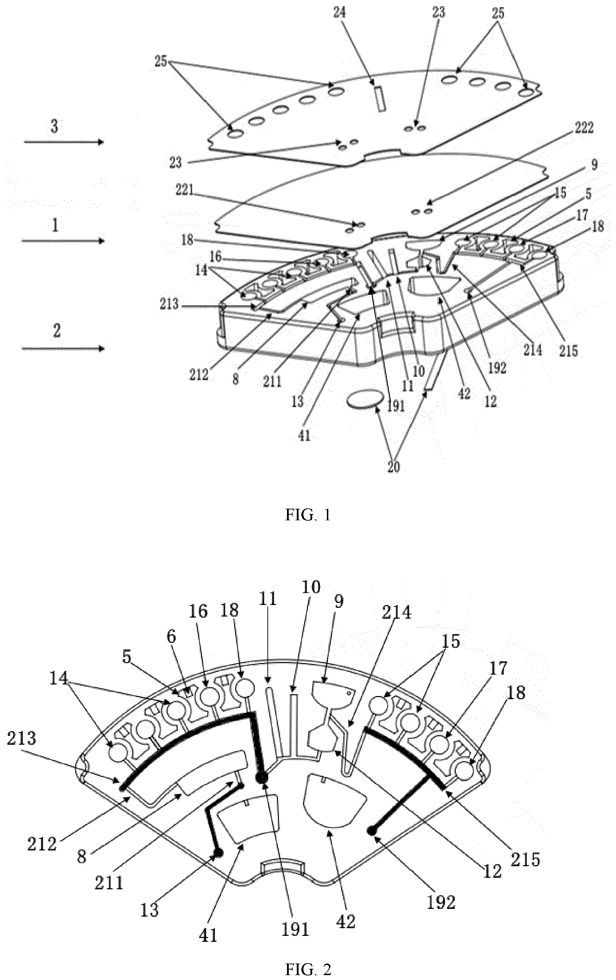

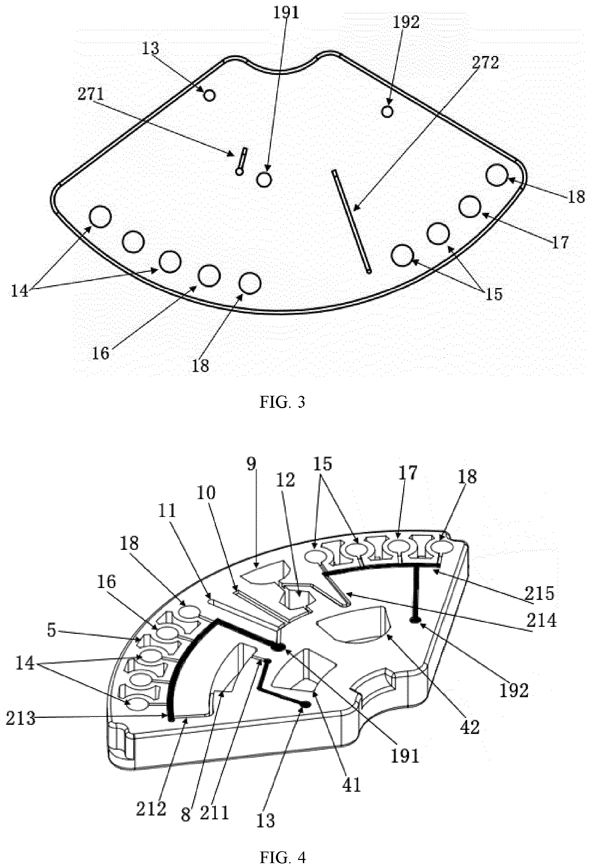

As shown in FIGS. 1-5, the microfluidic blood type detection chip in the present invention comprises a chip upper layer 1 and a chip body 2 serving as a lower chip.

As shown in FIG. 1, the chip upper layer 1 may be a transparent film, on which is provided with an upper-layer first sample injection chamber through hole 221 and an upper-layer second sample injection chamber through hole 222. The upper-layer first sample injection chamber through hole 221 and the upper-layer second sample injection chamber through hole 222 are used for adding whole blood samples, and are respectively located on the left and right sides of the chip upper layer 1 near the center.

As shown in FIGS. 1-5, the chip body 2 comprises: a forward typing blood type identification area, which is provided with a first sample injection chamber 41, a quantitative mixing chamber 8, a forward typing reaction chamber and a first vent hole 191; and a reverse typing blood type identification area, which is provided with a second sample injection chamber 42, a quantitative separation chamber, a sample overflow chamber 11, a reverse typing reaction chamber and a second vent hole 192.

The first sample injection chamber 41 and the second sample injection chamber 42 are respectively located on the left and right sides near the center of the chip body 2. The tops of the first sample injection chamber 41 and the second sample injection chamber 42 are both provided with injection ports. Specifically, the first sample injection chamber through hole 221 is located directly above the first sample injection chamber 41, and the second sample injection chamber through hole 222 is located directly above the second sample injection chamber 42. The reaction sample to be detected can be injected into each sample injection chamber through each sample injection chamber through hole.

The volume of the first sample injection chamber 41 is fixed and can be set to 100-200 µL, while the sample injection volume is 5-20 µL. The first sample injection chamber 41 is preset with a diluent, and is connected to the quantitative mixing chamber 8 through a microfluidic channel, and the quantitative mixing chamber 8 is farther from the center of the chip body 2 than the first sample injection chamber 41. Therefore, when the chip body 2 is driven to rotate by centrifugation, the mixed sample and diluent in the first sample injection chamber 41 will flow toward the quantitative mixing chamber 8 due to centrifugal action. Specifically, it flows toward the quantitative mixing chamber 8 through bottom port of the first sample injection chamber 41, then through a first reverse flow channel 271, and then through a first microfluidic channel 211. The inlet of the first reverse flow channel 271 is located at the bottom of the side of the first sample injection chamber 41 close to the quantitative mixing chamber 8, the outlet of the first microfluidic channel 211 is located on the side of the quantitative mixing chamber 8 close to the center of the chip body 2, and the first reverse flow channel 271 is connected with the first microfluidic channel 211 through a vertical flow channel. The first reverse flow channel 271 is provided with a sealing film 20 to prevent the sample from flowing out. The sample is added into the first sample injection chamber 41 and mixed with the diluent initially; then the mixture of the sample and the diluent is further mixed during the flow into the quantitative mixing chamber 8. There is no need to perform reciprocating motion in the quantitative mixing chamber 8 for mixing, thereby avoiding the risk of the mixed liquid entering the microfluidic channel leading to the reaction chamber during the mixing process. The mixture of the sample and the diluent is fully mixed in the quantitative mixing chamber 8 to form a blood cell suspension for detection.

The volume of the second sample injection chamber 42 is fixed and can be set to 250-350 µL, while the sample injection volume is 100-200 µL. The second sample injection chamber 42 is connected to the quantitative separation chamber through a microfluidic channel. Preferably, the quantitative separation chamber includes a separation chamber I 9 and a separation chamber II 12, and the separation chamber II 12 and the separation chamber I 9 are sequentially farther from the center of the chip body 2 than the second sample injection chamber 42. Two ends of the separation chamber I 9 are respectively connected with the second sample injection chamber 42 and the separation chamber II 12 through a microfluidic channel. The microfluidic channel connected between the separation chamber I 9 and the separation chamber II 12 is relatively narrow, and the function is that when the plasma enters the reaction chamber after stratification, it can ensure that the plasma enters the reaction chamber, and blood cells will not enter the reaction chamber and cause interference to the detection results. The separation chamber II 12 is connected to the sample overflow chamber 11 through microfluidic channel on the other side, and the sample overflow chamber 11 is located on the side of the quantitative separation chamber close to the forward typing blood type identification area. Therefore, when the chip body 2 is driven to rotate by centrifugation, the sample in the second sample injection chamber 42 will flow through bottom port of the second sample injection chamber 42 and a second reverse flow channel 272 toward the separation chamber I 9 due to centrifugal action. The inlet of the second reverse flow channel 272 is located at the bottom of the second sample injection chamber 42 near the quantitative separation chamber, and the outlet of the second reverse flow channel 272 is located on the side of the separation chamber I 9 away from the center of the chip body 2. The second reverse flow channel 272 is provided with a sealing film 20 to prevent the sample from flowing out. The sample exceeding the capacity of the separation chamber I 9 enters the separation chamber II 12 and the sample overflow chamber 11 successively. In the quantitative separation chamber, blood cells and plasma are stratified, the lower layer of blood cells is deposited, and the upper layer of plasma is used for detection.

The reaction chamber is located inside the outer edge of the chip body 2, including a forward typing reaction chamber and a reverse typing reaction chamber, and the volume of the reaction chambers is 20-40 µL. The forward typing reaction chamber includes a plurality of equidistantly and evenly distributed blood type antibody reagent reaction cavities 14, a forward typing quality control reaction cavity 16 and an overflow tank 18 located at the end of flow channel. Each blood type antibody reagent reaction cavity 14 and the forward typing quality control reaction cavity 16 are respectively provided with a blood type antibody reagent required for reaction and a quality control blood type antibody reagent. The reverse typing reaction chamber includes a plurality of equidistantly and evenly distributed blood type red blood cell reagent reaction cavities 15, a reverse typing quality control reaction cavity 17 and an overflow tank 18 located at the end of flow channel. Each blood type red blood cell reagent reaction cavity 15 and the reverse typing quality control reaction cavity 17 are respectively provided with a blood type red blood cell reagent required for reaction and a quality control blood type red blood cell reagent.

The quantitative mixing chamber 8 in the forward typing blood type identification area is connected to the forward typing reaction chamber through a second microfluidic channel 212 and a third microfluidic channel 213 successively. The quantitative separation chamber in the reverse typing blood type identification area is connected to the reverse typing reaction chamber through a fourth microfluidic channel 214 and a fifth microfluidic channel 215. The inlet of the fourth microfluidic channel 214 is located on the upper surface of microfluidic channel between separation chamber I 9 and separation chamber II 12 on the side close to the reverse typing blood type identification area. The flow channel height of the fourth microfluidic channel 214 is 1/5-1/2, preferably 1/5-1/3 of that of the microfluidic channel between the separation chamber I 9 and the separation chamber II 12, which further ensures that after the blood sample is stratified in the quantitative separation chamber, it is plasma that enters the reaction chamber, and blood cells will not enter the reaction chamber to affect the detection results. The blood cell suspension in the quantitative mixing chamber 8 and the plasma in the quantitative separation chamber can respectively enter the corresponding reaction cavities through microfluidic channels so as to react with the reaction reagents therein for detection. The excess blood cell suspension and plasma enter the corresponding overflow tanks 18 respectively. A single chip can simultaneously complete the blood type detection of two different separation reaction systems of forward and reverse typing, and the identification results of the two can be mutually verified, making the blood type identification results more accurate. It is easy to find and correct the wrong identification of blood type when the forward and reverse typing blood type detection results are inconsistent.

As shown in FIGS. 1-5, the quantitative mixing chamber 8, the forward typing reaction chamber and the reverse typing reaction chamber are respectively communicated with the first vent hole 191 and the second vent hole 192 each through a microfluidic channel. The vent holes penetrate through the chip body 2 located at the lower layer of the chip, and the configuration of vent holes makes the liquid flow in the chip more smoothly.

The sample overflow chamber 11 is communicated with the first vent hole 191 through a microfluidic channel. The first vent hole 191 can simultaneously serve as the vent hole of the forward typing blood type identification area and the reverse typing blood type identification area, which simplifies the chip structure and makes the flow of sample in the chip more smoothly.

The microfluidic channel between the first sample injection chamber 41 and the quantitative mixing chamber 8, the microfluidic channel between the quantitative mixing chamber 8 and the forward typing reaction chamber, and the microfluidic channel between the quantitative separation chamber and the reverse typing reaction chamber can be pre-filled with a solid phase change material, preferably a single-component alkane solid phase change material. As shown in FIG. 2, the filling of the solid phase change material can make the microfluidic channel between the first sample injection chamber 41 and the quantitative mixing chamber 8, the microfluidic channel between the quantitative mixing chamber 8 and the forward typing reaction chamber, and the microfluidic channel between the quantitative separation chamber and the reverse typing reaction chamber, always in a closed state when the chip is not in use, to encapsulate and protect the liquid reagents in the chip body 2. During the reaction, by adjusting the temperature in instrument detection chamber to reach a suitable range, the phase change material pre-filled in the microfluidic channel undergoes a phase change due to the temperature change in instrument detection chamber. Specifically, it changes from solid state to liquid state, so that the flow of liquid reagents in the chip can be realized. The phase change material that becomes liquid during the reaction enters the chamber. When the liquid reagent or sample enters, the phase change material will return to the microfluidic channel due to its small specific gravity. It stratifies with the lower liquid, and especially acts as an oil seal for the reaction chamber, thereby avoiding the volatilization of the reaction liquid in the reaction chamber to form bubbles and further affect the accuracy of the detection results. In addition, due to the oil seal of the phase change material, the reaction waste liquid in the chip will not leak after detection, which effectively reduces the risk of biological safety. More importantly, the use of phase change material encapsulation can well maintain the integrity of the blood cell reagent in the reverse typing reaction chamber, which solves the problem that the blood cell is easily broken when the blood type red blood cell reagent is lyophilized or dried.

The microfluidic blood type detection chip of the present invention further comprises a phase change material filling port, including the first phase change material filling port 13, the second phase change material filling port and the third phase change material filling port, which are connected to the quantitative mixing chamber 8, the forward typing reaction chamber and the reverse typing reaction chamber respectively through microfluidic channel. Preferably, the phase change material filling port can be a vent hole, and the aforementioned first vent hole 191 and second vent hole 192 can also be used as the second phase change material filling port and the third phase change material filling port. During chip production, after the upper sealing film is sealed to the chip body 2, the single-component alkane liquid phase change material is injected from the back of the chip body 2 through the first phase change material filling port 13, the second phase change material filling port and the third phase change material filling port. Due to the capillary force, the phase change material enters the flow channel to be sealed, so that the microfluidic channel between the first sample injection chamber 41 and the quantitative mixing chamber 8, the third microfluidic channel 213 between the quantitative mixing chamber 8 and the forward typing reaction chamber, and the fifth microfluidic channel 215 between the quantitative separation chamber and the reverse typing reaction chamber, are all pre-filled with a single-component alkane solid phase change material.

The microfluidic blood type detection chip of the present invention -may further comprises a hematocrit detection tube groove 10, which is located on the microfluidic channel between the quantitative separation chamber and the sample overflow chamber 11 in the reverse typing blood type identification area. It is a radial straight tube groove, and the marking scale on the surface of the tube groove is divided into 10 equal intervals, and each segment is further subdivided. Specifically, the effect of standard centrifugal force on overflowing blood can be used to directly detect hematocrit, and then manually interpret the hematocrit percentage, thereby providing an important basis for blood matching and supply (reference range of normal hematocrit value: female: 37% to 48%; male: 40% to 50%).

The microfluidic blood type detection chip of the present invention may further comprise anti-interference grooves 5 located between the reaction cavities. The anti-interference grooves 5 are used to prevent the light interference of two adjacent reaction cavities during the microscopic camera detection, which can affect the accuracy of the detection results. There is a positioning structure 6 inside each anti-interference groove 5, and the positioning structure 6 is a triangular column structure inside the anti-interference groove, which plays the role of detection and positioning during detection in the matching detection instrument.

Preferably, as shown in FIG. 1, the microfluidic blood type detection chip in the present invention may further comprise an opaque film 3, which is covered on the upper surface of the chip upper layer 1 and functions as an observation window. The opaque film 3 is provided with a plurality of through holes, and the through holes are arranged in order from near to far compared to the center of the opaque film 3: a sample injection chamber window 23, a hematocrit detection tube groove window 24 and reaction chamber windows 25. The sample injection chamber window 23 corresponds to the upper-layer first sample injection chamber through hole 221 and the upper-layer second sample injection chamber through hole 222 in the chip upper layer 1; the hematocrit detection tube groove window 24 corresponds to the hematocrit detection tube groove 10 on the chip body 2; and the reaction chamber windows 25 are in one-to-one correspondence with the reaction cavities on the chip body 2. When detecting, the staff can record the sample number and related information on the opaque film 3, without the need to attach labels, which is convenient and practical.

In the microfluidic blood type detection chip of the present invention, a diluent is preset in the second sample injection chamber 42 of the reverse typing blood type identification area, and the blood sample is diluted after being added, and then enters the subsequent detection process.

In the microfluidic blood type detection chip of the present invention, during chip production, the addition of the phase change material can also be carried out in the following ways. Before the transparent sealing film of the chip upper layer 1 is sealed to the chip body 2, and after the sealing film 20 is sealed to the chip body 2, reverse typing blood type identification area: the single-component alkane liquid phase change material is injected through the outlet of the second reverse flow channel 272, and the diluent is sealed in the second sample injection chamber 42 along the second reverse flow channel 272; chip reaction chamber: the blood type antibody reagent reaction cavities 14, the blood type red blood cell reagent reaction cavities 15, the forward typing quality control reaction cavity 16, and the reverse typing quality control reaction cavity 17, before the transparent sealing film of the chip upper layer 1 is sealed to the chip body 2, and after adding the reaction reagent to the reaction chamber, the single-component alkane liquid phase change material is directly added, and the change in temperature changes the phase change material from liquid to solid, sealing the microfluidic channel into the reaction chamber; forward typing blood type identification area: the single-component alkane liquid phase change material is injected through the vertical liquid inlet channel of the first microfluidic channel 211, and the diluent is sealed in the first sample injection chamber 41 along the first reverse flow channel 271.

The specific steps of using the microfluidic blood type detection chip of the present invention are as follows. A whole blood sample enters the first sample injection chamber 41 and the second sample injection chamber 42 through the upper-layer first sample injection hole 221 and the upper-layer second sample injection hole 222, respectively. The chip of the present invention is placed in a matching detection instrument, and according to detection requirements, the instrument is made to operate according to the set program. Forward typing blood type identification area: The phase change material pre-filled in the microfluidic channel between the first sample injection chamber 41 and the quantitative mixing chamber 8 changes from solid to liquid due to the temperature change in instrument detection chamber. Under centrifugation, the mixed sample and liquid diluent in the first sample injection chamber 41 flow out, and flow into the quantitative mixing chamber 8 through microfluidic channel. The mixture of the sample to be detected and the diluent is fully mixed in the flow process and in the quantitative mixing chamber 8 to form a blood cell suspension. The phase change material pre-filled in the third microfluidic channel 213 also changes from solid to liquid due to the temperature change in instrument detection chamber. Under centrifugation, the blood cell suspension in the quantitative mixing chamber 8 enters the forward typing reaction chamber through the second microfluidic channel 212 and the third microfluidic channel 213 successively; mixes with each anti-A antibody reagent, anti-B antibody reagent, and anti-D antibody reagent in blood type antibody reagent reaction cavities 14, as well as the quality control antibody reagent that does not contain anti-A, anti-B, and anti-D in the forward typing quality control reaction cavity 16, respectively, and reacts sufficiently; and the excess blood cell suspension flows into the overflow tank 18.

Reverse typing blood type identification area: during the reaction process of the sample entering the reaction chamber in the forward typing blood type identification area, under centrifugation, the sample in the second sample injection chamber 42 flows into the separation chamber I 9, the separation chamber II 12, and the hematocrit detection tube groove 10 successively through microfluidic channels, and the excess sample flows into the sample overflow chamber 11. Then, under the action of highspeed centrifugation, the blood cells and plasma are stratified in the separation chamber I 9 and the separation chamber II 12, the blood cells are deposited in the lower layer, and the upper layer is plasma. The phase change material pre-filled in the fifth microfluidic channel 215 changes from solid to liquid due to the temperature change in instrument detection chamber. Under centrifugation, the upper plasma in the quantitative separation chamber enters the reverse typing reaction chamber through the fourth microfluidic channel 214 and the fifth microfluidic channel 215 successively; mixes with each type A red blood cell reagent and type B red blood cell reagent in the blood type red blood cell reagent reaction cavities 15, as well as quality control red blood cell reagent (usually type a red blood cell reagent) that does not contain A and B blood type red blood cells in the reverse typing quality control reaction cavity 17, respectively, and reacts sufficiently; and the excess plasma flows into the overflow tank 18.

After the reactions in the forward typing blood type identification area and the reverse typing blood type identification area are completed, a microscopic camera is used to photograph, and the blood type identification results are automatically analyzed and displayed. It can also be combined with manual image review to judge the results. The data can also be automatically transmitted to company platform, and platform experts can review the judgment results. The results of blood type identification are shown in Table 1 below:

| Forward typing blood type identification area | | | | Reverse typing blood type identification area | | | Blood type identification result |
|---|---|---|---|---|---|---|---|
| Anti-A antibody reagent | Anti-B antibody reagent | Anti-D antibody reagent | Quality control antibody reagent | Type A RBC reagent | Type B RBC reagent | Quality control (type O) RBC reagent | |
| + | - | + | - | - | + | - | Type A Rh(D)+ |
| | + | + | - | + | - | - | Type B Rh(D)+ |
| + | + | + | - | - | - | - | Type AB Rh(D)+ |
| - | - | + | - | + | + | - | Type O Rh(D)+ |
| + | - | - | - | - | + | - | Type A Rh(D)- |
| - | + | - | - | + | - | - | Type B Rh(D)- |
| + | + | - | - | - | - | - | Type AB Rh(D)- |
| - | - | - | - | + | + | - | Type O Rh(D)- |

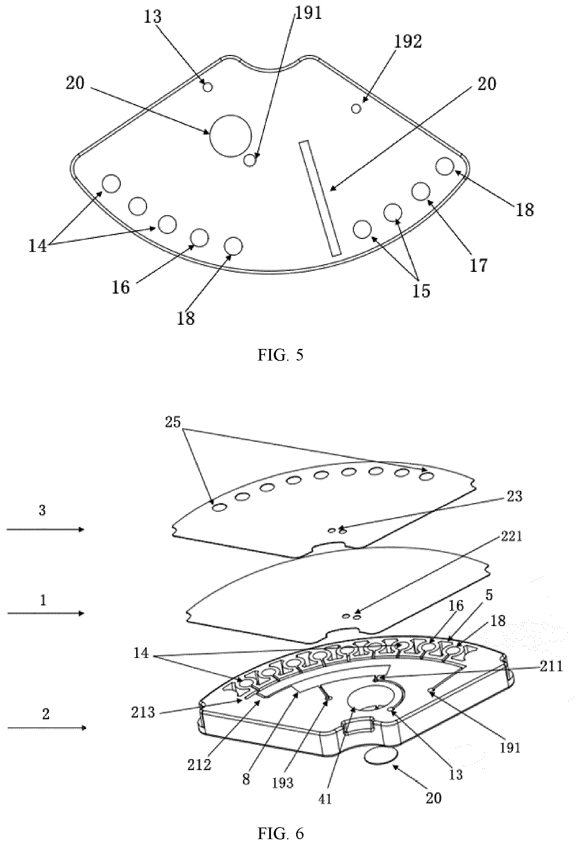

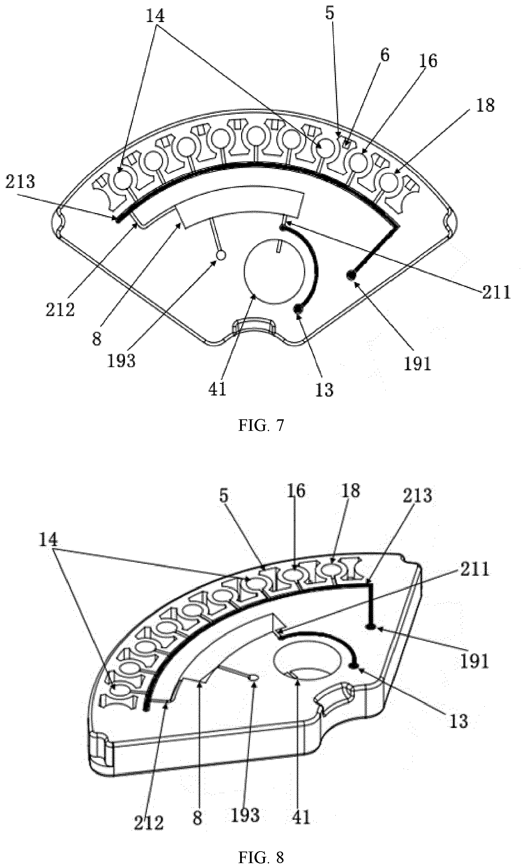

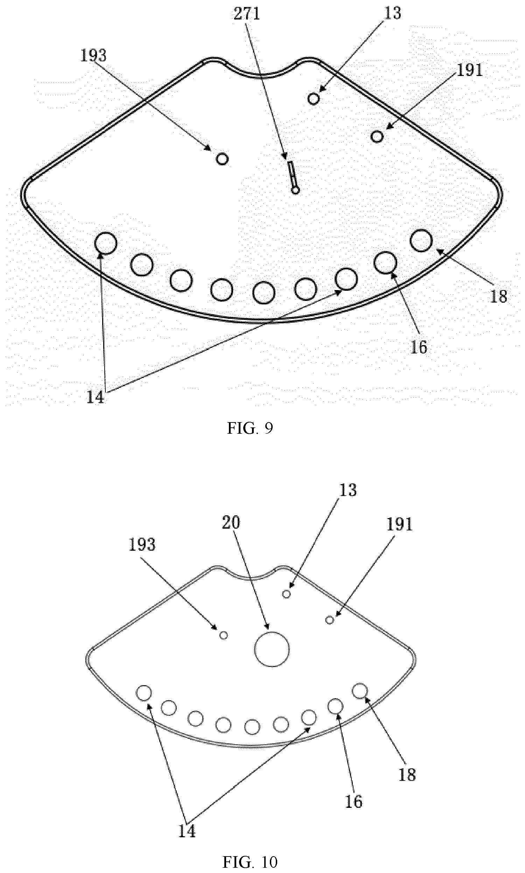

In another embodiment, not part of the present invention, as shown in FIGS. 6-10, it is a forward typing combined detection chip for ABO blood type and Rh blood type system. The quantitative mixing chamber 8 is connected to the third vent hole 193 through another microfluidic channel, and the configuration of the vent hole makes the liquid flow in the chip more smoothly. The chip is especially suitable for blood type identification of newborns. This is due to the lack of the newborn' own blood type antigen sites; and the low antibody titer. It is generally believed that there are basically no blood type antibodies within three months, and the detectable antibodies in the serum are often IgG blood type antibodies from the mother, which will lead to false positive results in the identification of neonatal reverse typing blood type. Therefore, the use of reverse typing to detect antibodies in serum of newborns is of little significance, and generally only forward typing blood type identification is done.

Specific steps are as follows. The whole blood sample enters the first sample injection chamber 41 through the upper-layer first sample injection chamber through hole 221. The chip of the present invention is placed in a detection instrument, and according to detection requirements, the instrument is made to operate according to the set program. The phase change material pre-filled in the microfluidic channel between the first sample injection chamber 41 and the quantitative mixing chamber 8 changes from solid to liquid due to the temperature change in instrument detection chamber. Under centrifugation, the mixed sample and liquid diluent in the first sample injection chamber 41 flow out, and flow into the quantitative mixing chamber 8 through microfluidic channel. The sample and the diluent are fully mixed in the flow process and in the quantitative mixing chamber 8 to form a blood cell suspension. The phase change material pre-filled in the third microfluidic channel 213 also changes from solid to liquid due to the temperature change in instrument detection chamber. Under centrifugation, the blood cell suspension in the quantitative mixing chamber 8 enters the forward typing reaction chamber through the second microfluidic channel 212 and the third microfluidic channel 213 successively; mixes with each anti-A antibody reagent, anti-B antibody reagent, anti-D antibody reagent, anti-C antibody reagent, anti-c antibody reagent, anti-E antibody reagent, and anti-e antibody reagent in blood type antibody reagent reaction cavities 14, as well as the quality control blood type antibody reagent that does not contain above blood type antibodies in the forward typing quality control reaction cavity 16, respectively, and reacts sufficiently; and the excess blood cell suspension flows into the overflow tank 18. A microscopic camera is used to photograph, and the blood type identification results are automatically analyzed and displayed. It can also be combined with manual image review to judge the results. The data can also be automatically transmitted to company platform, and platform experts can review the judgment results.

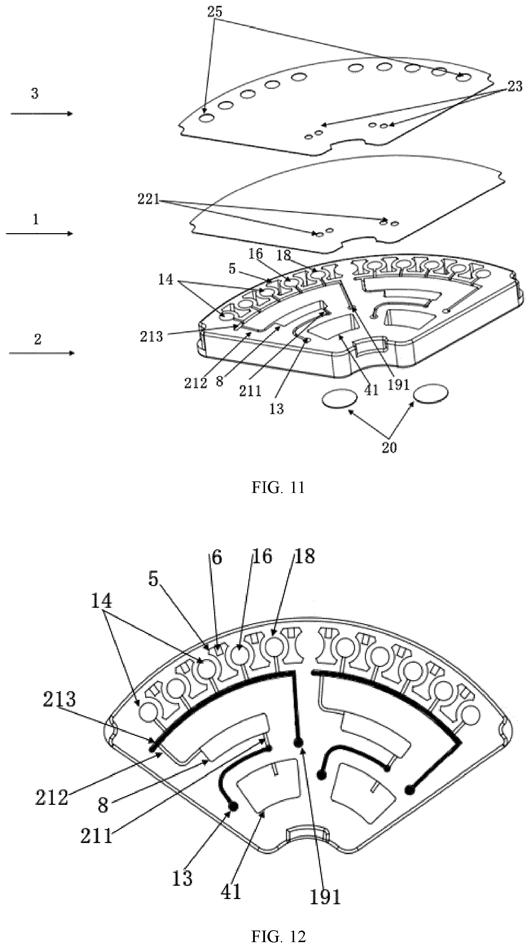

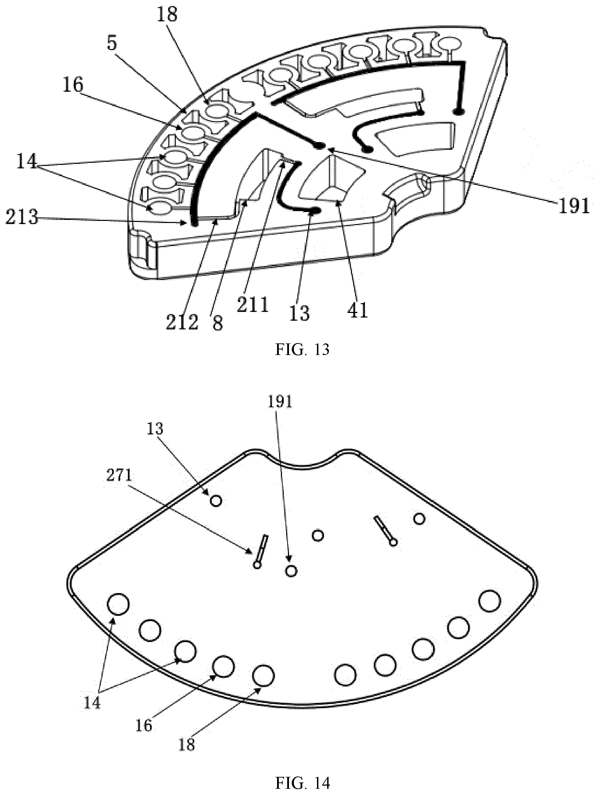

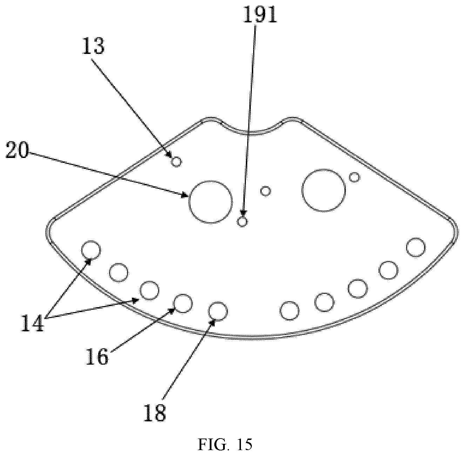

In yet another embodiment, not part of the present invention, as shown in FIGS. 11-15, two groups of ABO forward typing blood type identification areas can be set on the chip body 2 at the same time, that is, a double ABO forward typing blood type detection chip can be formed, which can detect two different samples at the same time. In addition, three chip bodies 2 can be assembled into a circular detection chip, which can detect six different samples at the same time, thereby increasing the throughput of the detection samples.

Specific steps are as follows. The whole blood sample enters the first sample injection chamber 41 through the upper-layer first sample injection chamber through hole 221. The chip of the present invention is placed in a detection instrument, and according to detection requirements, the instrument is made to operate according to the set program. The phase change material pre-filled in the microfluidic channel between the first sample injection chamber 41 and the quantitative mixing chamber 8 changes from solid to liquid due to the temperature change in instrument detection chamber. Under centrifugation, the mixed sample and liquid diluent in the first sample injection chamber 41 flow out, and flow into the quantitative mixing chamber 8 through microfluidic channel. The sample and the diluent are fully mixed in the flow process and in the quantitative mixing chamber 8 to form a blood cell suspension. The phase change material pre-filled in the third microfluidic channel 213 also changes from solid to liquid due to the temperature change in instrument detection chamber. Under centrifugation, the blood cell suspension in the quantitative mixing chamber 8 enters the forward typing reaction chamber through the second microfluidic channel 212 and the third microfluidic channel 213 successively; mixes with each anti-A antibody reagent, anti-B antibody reagent, and anti-D antibody reagent in blood type antibody reagent reaction cavities 14, as well as the quality control blood type antibody reagent that does not contain anti-A, anti-B, and anti-D in the forward typing quality control reaction cavity 16, respectively, and reacts sufficiently; and the excess blood cell suspension flows into the overflow tank 18. A microscopic camera is used to photograph, and the blood type identification results are automatically analyzed and displayed. It can also be combined with manual image review to judge the results. The data can also be automatically transmitted to company platform, and platform experts can review the judgment results.

The volume of the first sample injection chamber 41 in the forward typing blood type identification area of the microfluidic blood type detection chip of the present invention is 100-200 µL, the sample injection volume is 5-20 µL, and the blood collection volume is small. Therefore, the problem of applicability for people who have difficulty in blood collection, especially for newborns, can be well solved.

The above descriptions are only preferred embodiments of the present invention, and are not intended to limit the present invention. The scope of the invention is defined by the claims.

## Claims

1. A microfluidic blood type detection chip, the chip comprises a chip body (2), and the chip body (2) comprises: a forward typing blood type identification area, which is provided with a first sample injection chamber (41), a quantitative mixing chamber (8), and a forward typing reaction chamber; **characterized in that**,
a reverse typing blood type identification area, which is provided with a second sample injection chamber (42), a quantitative separation chamber, and a reverse typing reaction chamber; wherein the quantitative separation chamber is configured to stratify
plasma sample and blood cell sample from the sample to be detected;
vent holes communicated with the forward typing reaction chamber and the reverse typing reaction chamber respectively;
the first sample injection chamber (41) and the second sample injection chamber (42) are used for injecting a sample to be detected, and are respectively connected with the quantitative mixing chamber (8) and the quantitative separation chamber through a microfluidic channel, the reaction sample enters the quantitative mixing chamber (8) and the quantitative separation chamber respectively from the first sample injection chamber (41) and the second sample injection chamber (42);
a diluent is preset in the first sample injection chamber (41), the diluent is configured to enter the quantitative mixing chamber (8) through the first sample injection chamber (41);
a diluent is preset in the second sample injection chamber (42), the diluent is configured to enter the quantitative separation chamber through the second sample injection chamber (42);
the forward typing reaction chamber is configured for, when the reaction sample and the diluent in the first sample injection chamber (41) are mixed in the quantitative mixing chamber (8), the blood cell sample enters the forward typing reaction chamber through a microfluidic channel to react with reaction reagent therein for detection;
the reverse typing reaction chamber is configured for, the plasma sample separated from the quantitative separation chamber enters the reverse typing reaction chamber through a microfluidic channel to react with reaction reagent therein for detection;
a hematocrit detection tube groove (10), which is connected to the quantitative separation chamber and is located on the side of the quantitative separation chamber close to the forward typing blood type identification area.

2. The microfluidic blood type detection chip according to claim 1, **characterized in that**, the forward typing reaction chamber includes a plurality of blood type antibody reagent reaction cavities, a forward typing quality control reaction cavity (16) and an overflow tank (18);
the reverse typing reaction chamber includes a plurality of blood type red blood cell reagent reaction cavities, a reverse typing quality control reaction cavity (17) and an overflow tank (18).

3. The microfluidic blood type detection chip according to claim 2, **characterized in that**, the plurality of blood type antibody reagent reaction cavities and the plurality of blood type red blood cell reagent reaction cavities are respectively equidistantly distributed.

4. The microfluidic blood type detection chip according to claim 1, **characterized in that**, the reverse typing blood type identification area further comprises a sample overflow chamber (11), which is connected with the quantitative separation chamber, and the reaction sample exceeding the quantitative separation chamber enters the sample overflow chamber (11).

5. The microfluidic blood type detection chip according to claim 4, **characterized in that**, the quantitative separation chamber includes a separation chamber I (9) and a separation chamber II (12), the sample overflow chamber (11) and the separation chamber I (9) are respectively connected to the separation chamber II (12) through different microfluidic channels, the reaction sample exceeding the separation chamber I (9) enters the separation chamber II (12) and the sample overflow chamber (11) successively.

6. The microfluidic blood type detection chip according to claim 4, **characterized in that**, the sample overflow chamber (11) is connected with the vent hole that is communicated with the forward typing reaction chamber.

7. The microfluidic blood type detection chip according to claim 1, **characterized in that**, the microfluidic channel between the first sample injection chamber (41) and the quantitative mixing chamber (8), the microfluidic channel between the quantitative mixing chamber (8) and the forward typing reaction chamber, and the microfluidic channel between the quantitative separation chamber and the reverse typing reaction chamber are pre-filled with a solid phase change material.

8. The microfluidic blood type detection chip according to claim 7, **characterized in that**, the phase change material is a single-component alkane solid phase change material.

9. The microfluidic blood type detection chip according to claim 7, **characterized in that**, the chip further comprises a phase change material filling port.

10. The microfluidic blood type detection chip according to claim 9, **characterized in that**, the phase change material filling port is a vent hole.

11. The microfluidic blood type detection chip according to claim 1, **characterized in that**, the volumes of the forward typing reaction chamber and the reverse typing reaction chamber are both 20-40 µL.

12. The microfluidic blood type detection chip according to claim 1, **characterized in that**, the volume of the first sample injection chamber (41) is 100-200 µL.

13. The microfluidic blood type detection chip according to claim 1, **characterized in that**, the volume of the second sample injection chamber (42) is 250-350 µL.

## Patentansprüche

1. Ein mikrofluidischer Blutgruppenerkennungschip, der einen Chipkörper (2) umfasst, und der Chipkörper (2) umfasst: einen Vorwärtstypisierungs-Blutgruppenidentifizierungsbereich, der mit einer ersten Probeninjektionskammer (41), einer quantitativen Mischkammer (8) und einer Vorwärtstypisierungsreaktionskammer versehen ist; **dadurch gekennzeichnet, dass**,
einen Bereich zur Blutgruppenidentifizierung mit umgekehrter Typisierung, der mit einer zweiten Probeninjektionskammer (42), einer quantitativen Trennkammer und einer Reaktionskammer zur umgekehrten Typisierung ausgestattet ist; wobei die quantitative Trennkammer so konfiguriert ist, dass sie Plasmaproben und Blutzellenproben von der zu erkennenden Probe trennt;
Entlüftungslöcher, die mit der Vorwärtstypisierungsreaktionskammer bzw. der Rückwärtstypisierungsreaktionskammer verbunden sind;
die erste Probeninjektionskammer (41) und die zweite Probeninjektionskammer (42) dienen zum Injizieren einer zu erfassenden Probe und sind jeweils durch einen Mikrofluidkanal mit der quantitativen Mischkammer (8) und der quantitativen Trennkammer verbunden, wobei die Reaktionsprobe aus der ersten Probeninjektionskammer (41) und der zweiten Probeninjektionskammer (42) in die quantitative Mischkammer (8) bzw. die quantitative Trennkammer gelangt;
ein Verdünnungsmittel ist in der ersten Probeninjektionskammer (41) voreingestellt, das Verdünnungsmittel ist so konfiguriert, dass es durch die erste Probeninjektionskammer (41) in die quantitative Mischkammer (8) gelangt;
ein Verdünnungsmittel ist in der zweiten Probeninjektionskammer (42) voreingestellt, das Verdünnungsmittel ist so konfiguriert, dass es durch die zweite Probeninjektionskammer (42) in die quantitative Trennkammer gelangt;
die Vorwärtstypisierungsreaktionskammer ist so konfiguriert, dass, wenn die Reaktionsprobe und das Verdünnungsmittel in der ersten Probeninjektionskammer (41) in der quantitativen Mischkammer (8) gemischt werden, die Blutzellenprobe durch einen Mikrofluidkanal in die Vorwärtstypisierungsreaktionskammer gelangt, um mit dem darin enthaltenen Reaktionsreagenz zur Erkennung zu reagieren;
Die Reverse-Typing-Reaktionskammer ist so konfiguriert, dass die aus der quantitativen Trennkammer abgetrennte Plasmaprobe über einen Mikrofluidkanal in die Reverse-Typing-Reaktionskammer gelangt, um dort mit dem darin enthaltenen Reaktionsreagenz zur Erkennung zu reagieren;
eine Hämatokrit-Erkennungsröhrchennut (10), die mit der quantitativen Trennkammer verbunden ist und sich auf der Seite der quantitativen Trennkammer in der Nähe des Vorwärtstypisierungs-Blutgruppenidentifizierungsbereichs befindet.

2. Mikrofluidischer Blutgruppenbestimmungschip nach Anspruch 1, **dadurch gekennzeichnet, dass** die Vorwärtstypisierungsreaktionskammer eine Vielzahl von Blutgruppenantikörper-Reagenzreaktionskammern, eine Vorwärtstypisierungs-Qualitätskontroll-Reaktionskammer (16) und einen Überlauftank (18) umfasst;
Die Reaktionskammer für die umgekehrte Typisierung umfasst eine Vielzahl von Reaktionskammern für Reagenzien zur Bestimmung der roten Blutkörperchen, eine Reaktionskammer für die umgekehrte Typisierung zur Qualitätskontrolle (17) und einen Überlaufbehälter (18).

3. Mikrofluidischer Blutgruppen-Erkennungschip nach Anspruch 2, **dadurch gekennzeichnet, dass** die Vielzahl von Blutgruppen-Antikörper-Reagenz-Reaktionskammern und die Vielzahl von Blutgruppen-Erythrozyten-Reagenz-Reaktionskammern jeweils äquidistant verteilt sind.

4. Mikrofluidischer Blutgruppen-Erkennungschip nach Anspruch 1, **dadurch gekennzeichnet, dass** der Bereich zur umgekehrten Blutgruppenidentifizierung außerdem eine Probenüberlaufkammer (11) umfasst, die mit der quantitativen Trennkammer verbunden ist, und dass die Reaktionsprobe, die die quantitative Trennkammer überschreitet, in die Probenüberlaufkammer (11) gelangt.

5. Mikrofluidischer Blutgruppen-Erkennungschip nach Anspruch 4, **dadurch gekennzeichnet, dass** die quantitative Trennkammer eine Trennkammer I (9) und eine Trennkammer II (12) umfasst, die Probenüberlaufkammer (11) und die Trennkammer I (9) jeweils über verschiedene Mikrofluidkanäle mit der Trennkammer II (12) verbunden sind, wobei die Reaktionsprobe, die die Trennkammer I (9) überschreitet, nacheinander in die Trennkammer II (12) und die Probenüberlaufkammer (11) gelangt.

6. Mikrofluidischer Blutgruppen-Erkennungschip nach Anspruch 4, **dadurch gekennzeichnet, dass** die Probenüberlaufkammer (11) mit dem Entlüftungsloch verbunden ist, das mit der vorderen Typisierungsreaktionskammer in Verbindung steht.

7. Mikrofluidischer Blutgruppen-Erkennungschip nach Anspruch 1, **dadurch gekennzeichnet, dass** der Mikrofluidkanal zwischen der ersten Probeninjektionskammer (41) und der quantitativen Mischkammer (8), der Mikrofluidkanal zwischen der quantitativen Mischkammer (8) und der Vorwärtstypisierungsreaktionskammer und der Mikrofluidkanal zwischen der quantitativen Trennkammer und der Rückwärtstypisierungsreaktionskammer mit einem festen Phasenwechselmaterial vorgefüllt sind.

8. Mikrofluidischer Blutgruppenerkennungschip nach Anspruch 7, **dadurch gekennzeichnet, dass** das Phasenwechselmaterial ein einkomponentiges Alkan-Festphasenwechselmaterial ist.

9. Mikrofluidischer Blutgruppen-Erkennungschip nach Anspruch 7, **dadurch gekennzeichnet, dass** der Chip zusätzlich eine Einfüllöffnung für Phasenwechselmaterial aufweist.

10. Mikrofluidischer Blutgruppen-Erkennungschip nach Anspruch 9, **dadurch gekennzeichnet, dass** die Einfüllöffnung für das Phasenwechselmaterial eine Entlüftungsöffnung ist.

11. Der mikrofluidische Blutgruppenerkennungschip nach Anspruch 1, **dadurch gekennzeichnet, dass** das Volumen der Vorwärtstypisierungsreaktionskammer und der Rückwärtstypisierungsreaktionskammer beide 20-40 µL betragen.

12. Mikrofluidischer Blutgruppen-Erkennungschip nach Anspruch 1, **dadurch gekennzeichnet, dass** das Volumen der ersten Probeninjektionskammer (41) 100-200 µL beträgt.

13. Mikrofluidischer Blutgruppen-Erkennungschip nach Anspruch 1, **dadurch gekennzeichnet, dass** das Volumen der zweiten Probeninjektionskammer (42) 250-350 µL beträgt.

## Revendications

1. une puce microfluidique de détection du groupe sanguin, la puce comprend un corps de puce (2), et le corps de puce (2) comprend : une zone d'identification du groupe sanguin par typage direct, qui est pourvue d'une première chambre d'injection d'échantillon (41), d'une chambre de mélange quantitatif (8), et d'une chambre de réaction de typage direct ; **caractérisée par le fait que**,
une zone d'identification du groupe sanguin par typage inverse, qui est pourvue d'une seconde chambre d'injection d'échantillon (42), d'une chambre de séparation quantitative et d'une chambre de réaction de typage inverse ; la chambre de séparation quantitative étant configurée pour stratifier l'échantillon de plasma et l'échantillon de cellules sanguines à partir de l'échantillon à détecter ;
des trous d'aération communiquaient respectivement avec la chambre de réaction de typage direct et la chambre de réaction de typage inverse ;
la première chambre d'injection d'échantillon (41) et la seconde chambre d'injection d'échantillon (42) sont utilisées pour injecter un échantillon à détecter, et sont respectivement reliées à la chambre de mélange quantitative (8) et à la chambre de séparation quantitative par l'intermédiaire d'un canal microfluidique, l'échantillon de réaction entre dans la chambre de mélange quantitative (8) et dans la chambre de séparation quantitative respectivement depuis la première chambre d'injection d'échantillon (41) et la deuxième chambre d'injection d'échantillon (42) ;
un diluant est préréglé dans la première chambre d'injection d'échantillon (41), le diluant est configuré pour entrer dans la chambre de mélange quantitatif (8) à travers la première chambre d'injection d'échantillon (41) ;
un diluant est préréglé dans la seconde chambre d'injection d'échantillon (42), le diluant est configuré pour entrer dans la chambre de séparation quantitative à travers la seconde chambre d'injection d'échantillon (42) ;
la chambre de réaction de typage direct est configurée pour que, lorsque l'échantillon de réaction et le diluant dans la première chambre d'injection d'échantillon (41) sont mélangés dans la chambre de mélange quantitatif (8), l'échantillon de cellules sanguines pénètre dans la chambre de réaction de typage direct par un canal microfluidique pour réagir avec le réactif de réaction à l'intérieur de celle-ci pour la détection ;
la chambre de réaction de typage inverse est configurée pour que l'échantillon de plasma séparé de la chambre de séparation quantitative pénètre dans la chambre de réaction de typage inverse par un canal microfluidique pour réagir avec le réactif de réaction à l'intérieur pour la détection ;
une rainure de tube de détection d'hématocrite (10), qui est reliée à la chambre de séparation quantitative et est située sur le côté de la chambre de séparation quantitative à proximité de la zone d'identification du groupe sanguin de typage direct.

2. La puce microfluidique de détection de groupe sanguin selon la revendication 1, **caractérisée par le fait que** la chambre de réaction de typage direct comprend une pluralité de cavités de réaction de réactif d'anticorps de groupe sanguin, une cavité de réaction de contrôle de qualité de typage direct (16) et un réservoir de trop-plein (18) ;
la chambre de réaction de typage inverse comprend une pluralité de cavités de réaction de réactif de globules rouges de type sanguin, une cavité de réaction de contrôle de qualité de typage inverse (17) et un réservoir de trop-plein (18).

3. La puce de détection de type sanguin microfluidique selon la revendication 2, **caractérisée par le fait que** la pluralité de cavités de réaction de réactif d'anticorps de type sanguin et la pluralité de cavités de réaction de réactif de globules rouges de type sanguin sont respectivement réparties de manière équidistante.

4. La puce de détection de groupe sanguin microfluidique selon la revendication 1, **caractérisée par le fait que** la zone d'identification de groupe sanguin de typage inverse comprend en outre une chambre de débordement d'échantillon (11), qui est reliée à la chambre de séparation quantitative, et l'échantillon de réaction dépassant la chambre de séparation quantitative entre dans la chambre de débordement d'échantillon (11).

5. La puce microfluidique de détection de groupe sanguin selon la revendication 4, **caractérisée par le fait que** la chambre de séparation quantitative comprend une chambre de séparation I (9) et une chambre de séparation II (12), la chambre de débordement d'échantillon (11) et la chambre de séparation I (9) sont respectivement reliées à la chambre de séparation II (12) par différents canaux microfluidiques, l'échantillon de réaction dépassant la chambre de séparation I (9) entre successivement dans la chambre de séparation II (12) et la chambre de débordement d'échantillon (11).

6. La puce de détection de groupe sanguin microfluidique selon la revendication 4, **caractérisée par le fait que** la chambre de débordement d'échantillon (11) est reliée au trou d'aération qui communique avec la chambre de réaction de typage direct.

7. La puce microfluidique de détection de groupe sanguin selon la revendication 1, **caractérisée par le fait que** le canal microfluidique entre la première chambre d'injection d'échantillon (41) et la chambre de mélange quantitatif (8), le canal microfluidique entre la chambre de mélange quantitatif (8) et la chambre de réaction de typage direct, et le canal microfluidique entre la chambre de séparation quantitative et la chambre de réaction de typage inverse sont pré-remplis d'un matériau à changement de phase solide.

8. La puce de détection de groupe sanguin microfluidique selon la revendication 7, **caractérisée par le fait que** le matériau à changement de phase est un matériau à changement de phase solide alcane monocomposant.

9. La puce de détection de groupe sanguin microfluidique selon la revendication 7, **caractérisée en ce que** la puce comprend en outre un orifice de remplissage en matériau à changement de phase.

10. La puce de détection de groupe sanguin microfluidique selon la revendication 9, **caractérisée par le fait que** l'orifice de remplissage du matériau à changement de phase est un trou d'aération.

11. La puce de détection de groupe sanguin microfluidique selon la revendication 1, **caractérisée par le fait que** les volumes de la chambre de réaction de typage direct et de la chambre de réaction de typage inverse sont tous deux de 20 à 40 µL.

12. La puce de détection de groupe sanguin microfluidique selon la revendication 1, **caractérisée par le fait que** le volume de la première chambre d'injection d'échantillon (41) est de 100 à 200 µL.

13. La puce de détection de groupe sanguin microfluidique selon la revendication 1, **caractérisée par le fait que** le volume de la seconde chambre d'injection d'échantillon (42) est de 250 à 350 µL.
